(19) Europäisches Patentamt
European Patent Office
Office européen des brevets

(11) EP 1 276 874 B1

(12) # FASCICULE DE BREVET EUROPEEN

(45) Date de publication et mention de la délivrance du brevet:
**03.10.2007 Bulletin 2007/40**

(21) Numéro de dépôt: **00925351.9**

(22) Date de dépôt: **28.04.2000**

(51) Int Cl.:
***C12N 15/55*** *(2006.01)* ***C12N 15/74*** *(2006.01)*
***C12N 15/79*** *(2006.01)* ***C12N 9/20*** *(2006.01)*

(86) Numéro de dépôt international:
**PCT/FR2000/001148**

(87) Numéro de publication internationale:
**WO 2001/083773 (08.11.2001 Gazette 2001/45)**

(54) **CLONAGE ET EXPRESSION D'UNE LIPASE EXTRACELLULAIRE ACIDORESISTANTE DE YARROWIA LIPOLYTICA**

KLONIERUNG UND EXPRESSION EINER EXTRAZELLULÄREN SÄUREBESTÄNDIGEN LIPASE AUS YARROWIA LIPOLYTICA

CLONING AND EXPRESSING AN ACID-RESISTANT EXTRACELLULAR LIPASE OF YARROWIA LIPOLYTICA

(84) Etats contractants désignés:
**AT BE CH CY DE DK ES FI FR GB GR IE IT LI LU MC NL PT SE**

(43) Date de publication de la demande:
**22.01.2003 Bulletin 2003/04**

(73) Titulaires:
- **LABORATOIRES MAYOLY SPINDLER**
  **78401 Chatou Cédex (FR)**
- **Seman, Michel**
  **F-75003 Paris (FR)**

(72) Inventeurs:
- **SEMAN, Michel**
  **F-75003 Paris (FR)**
- **PIGNEDE, Georges**
  **F-92270 Bois Colombes (FR)**
- **FUDALEJ, Franck**
  **F-92500 Rueil-Malmaison (FR)**
- **NICAUD, Jean-Marc**
  **F-78190 Trappes (FR)**
- **GAILLARDIN, Claude**
  **F-78000 Versailles (FR)**

(74) Mandataire: **Galup, Cédric Olivier Nicolas et al**
**Cabinet ORES,**
**36,rue de St Pétersbourg**
**75008 Paris (FR)**

(56) Documents cités:
- **EMBL Database, Heidelberg, FRG Emfun accession number Z49607 6 octobre 1995 RAMEZANI, R.M. ET AL.: "S. cerevisiae chromosome X reading frame ORF YJR107w." XP002160656 -& EMBL Database, Heidelberg, FRG Swissprot accession number P47145 1 février 1996 RAMEZANI, R.M. ET AL.: "HYPOTHETICAL LIPASE IN SOD1-CPA2 INTERGENIC REGION (3.1.1.-)." XP002160657 cité dans la demande**
- **NOVOTNY, C. ET AL.: "The production of lipases by some Candida and Yarrowia yeasts" JOURNAL OF BASIC MICROBIOLOGY, vol. 28, no. 4, 1988, pages 221-227, XP002105080 cité dans la demande**
- **PIGNÈDE, G. ET AL.: "Characterization of an Extracellular Lipase Encoded by LIP2 in Yarrowia lipolytica" JOURNAL OF BACTERIOLOGY , vol. 182, no. 10, mai 2000 (2000-05), pages 2802-2810, XP002160655**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 276 874 B1

LISTE DE SEQUENCES

<110> LABORATOIRES MAYOLY SPINDLER
SEMAN, Michel
PIGNEDE, Georges
FUDALEJ, Franck
NICAUD, Jean-Marc
GAILLARDIN, Claude

<120> CLONAGE ET EXPRESSION D'UNE LIPASE EXTRACELLULAIRE ACIDORESISTANTE DE LEVURE

<130> MJPcb269/24

<140>
<141>

<160> 2

<170> PatentIn Ver. 2.1

<210> 1
<211> 5304
<212> ADN
<213> Yarrowia lipolytica

<220>
<221> CDS
<222> (3737)..(4741)

<220>
<221> mat_peptide
<222> (3836) (4741)

<400> 1

gatatcggta gcaaacagac acgaagaaga ggccttggag aatttggaaa caacatccat 60
tcgtgctcct tgtttctgct tgccatgcaa atgcaacagg gggataccgg gctgcagagt 120
tcggaaggtc tcgtagacgt atcgcacctg ttttgatgaa ctgaagaaaa cgatgatctt 180
gaacttggtg tgtgttctca ggaaacccca cagtgtgtcc agcttgtctt gcagctccac 240
gcagacatag ttctgttcga ggttcttggg tgtgctggaa gtggtgtccg ggttggcgga 300
gatgtacttg gggtcggcga gtgacaaacg ggccagatca ctgacgctct tggtctgtgt 360
tgcgctgaaa agcagagtct gtctgtccac aggcaggttt tccaaaatgg catccatggt 420
cttcttgaaa cccatatcga ggattcgatc ggcctcgtcc agcaccagca tcttcacgtt 480
agaaagatca aacccgctag tttggtccat gtgttgtaac agtcgtccgg gtgtacaaat 540
gagaatgttc agcttggcca gtcggtcagc ctccatggca acgtcctttc ctccaataac 600
caaaccagca gagaagctgt gacaacggcc gatttttcgc agaacctgga aaatctggac 660
cgccagttct cgagtcggac taataaccag ggctcctaaa ccatccacat cactccactt 720
gtttcgaaac aggcactcca acacaggcac cagaaacgcc agagtcttac cacttccggt 780

acgcgctgct ccaagcagat cgtggccttg cagagcggga gggatggccc gcttctgaac 840
atcggtacag gtgacataat gtgaattttt tagaccctca atagtagcct cagacaaggg 900
cagctcctca aacttggaca cctccttgtc ggcctcgtcc tctccaaagt tgtcgacgcg 960
ctcctgcagc ttggagagct tctgtttgtg ttcctctcgt ttgattttac gaggatcggc 1020
tcgtttcttc attttcgcca tatctcaagc tgtcggataa atttcttgga caaaaaaatc 1080
taacttttcg tgcgccttca cttttatcag tctgaaaatt acacatgcat gcagcttgga 1140
gtatggaggc gacagaggtg catgccagag tgcatgaatt agggcaacgg gggtgtgaag 1200
aagggcgcaa aagatgtgac gacggtgcaa gacgacggta tactgcagac aaggtctttc 1260
actgcaaaac tcctggttaa atcgtgggtc ccgcgtctcc gtatcatatg actttgggtc 1320
acgtgactgc aagattttta aggtttcgaa ctcccaaatt gagtcacgtg tcgtctctct 1380
gcaacatgag gtatcgtaaa ttaggaatga ctcgacctga caggccatca aggaccctga 1440
tgcaactctg aaattagagg gaggaggtaa tgagaaacag tgctggattg tggtgagaca 1500
ttgaagcggg attggttaac ctgtttcttt tttgtcgttt gctcttcatt attgcgattt 1560
ttgtagtcat ttgattagat cagatcagca gtcccctcat gctgtcgttg atgggcgcgt 1620
aatcagtgta acgctttgaa tgaggatggt gtgagtaata ctggtttcat tggggggggg 1680
gggggggta ctgttactcg aaaaacaaat catatgcatc tctgaacgtg ccggggtgtg 1740
gcatgtttct cgcttcgaaa aataaatgtt ggctgttcca ggcttgtctc agacctgcaa 1800
gaatcatact ggcgtaatct ccgccaggtc tggtcaggaa caagagagta aaagaaatgt 1860
ttgagtctcg gctttcacat cctcggcact agataagcag caatatcaat gtcacattgc 1920
ttgcttgtgc aggtcagggg gaggggggatg tatatgatac aagcaggttc gcatctacag 1980
aatctacagt atgtatctga tgatagcctt acacagcgca cgcagatgta gtttttgggt 2040
tgaacagtgt cagcggaagc ggcgtagtgg ggtactgtag tacagaatca acggaatgaa 2100
gtttacaact gcttctgtta ctgtagcctt tcactgttca aatgataacg ttggagaaga 2160
gtgcaccctc tgttagatag gattacagtc cagacacaca ttggcaccga agtctgtagt 2220
caaacattgg tctcagttgg tgtaatgaca ttcaatagac tgattctgca actcctacac 2280
cttgcattgt acacccgaga ccccttgtac ggcccgcatt tctccttcac atgtcggcat 2340
gtgtcttgtc tgtagccact tcaatgggat gcagtattag cactgtcgga cacacctcag 2400
tcaatgggaa tggaatgcca tgctcctagc ggctcattgt ttgctaggaa atacagttaa 2460
tagcacagat catagagtct aggctctata cgtcttagaa caaccattat ttgtttaaac 2520
gacaacttgg taagcgaatt acagtaagta gctattgttt ctatctctgt cagtgttcgg 2580
ataagtcctt agatcaccat actcaaagtg gtacgtttcg tcgcatcgga cgagaagtgt 2640

```
gtctatcacc gtttcttttt gccgctctca tttttgcggt cggaataatt actgtggacc 2700
ctcggcgccg accgttttgc agggctcatc aacctaaaac ttctccgagt ctgtgccttc 2760
aggtgggcat agttgatggg tgttttgaag ttaatagtgg ggaagaacta tggcaaacaa 2820
gcagatgcag gcaccttgta actgcagacc ggttcttgtc taccgactcc gctgcacctg 2880
tgccgcggta catgtcgtca caggctgcgg ggttcggagg ccccccttgca acctcctttg 2940
atagttgcta tggccccaaa gagttatacg agatagaccc acagatctac ttgactgttg 3000
tcacagaacc tgctaggttt gcttattgta cccgctttgt agctactgta caacgacaac 3060
gtctaaaatt gagacgcgaa caaactccag atgcagaacc caaacctctc tctcagagtt 3120
tcgagtgctt ctacctcaca gtaaagtgga ggtggacctg caagggaatt cagtcacaag 3180
gccccgaatg tctccgaaac tccaatcgga ccgtttaaac agactaatat cacgtcattg 3240
attgatatta gcatccggca agagccgcaa ggttatctcc tcaccaatga gcctgttgta 3300
cggctcattc cgcatctgcg gctgattcag tttcgagtgg ggatggtaga cttcattgca 3360
gcattcctaa ccttctactt ggtccgtgga gatgtcatgg acatcgattt tgggctgaga 3420
agccttttga cgatgtcgat atcactgacc gctaatttac tctggcagtt tctccggctc 3480
tcgaggcatc gtcgatcacc aaacactatc tgctagtcta aatgtccgac acgacagctt 3540
ttgatcgccg tgaacggcgc agacctcatg caccatgcac cagggccaaa tcaattacgg 3600
gtcgcttagc gttgcagtcg gggcattatg gtggaagttc cgatacggca cagacacatt 3660
ccatagtggg gggattggat tataaaaggg ccatagaaag ccctcaattg atacccaagt 3720
accagctccc ctcact atg aag ctt tcc acc atc ctt ttc aca gcc tgc gct 3772
                  Met Lys Leu Ser Thr Ile Leu Phe Thr Ala Cys Ala
                              -30                 -25

acc ctg gct gcc gcc ctc cct tcc ccc atc act cct tct gag gcc gca    3820
Thr Leu Ala Ala Ala Leu Pro Ser Pro Ile Thr Pro Ser Glu Ala Ala
    -20                 -15                 -10

gtt ctc cag aag cga gtg tac acc tct acc gag acc tct cac att gac    3868
Val Leu Gln Lys Arg Val Tyr Thr Ser Thr Glu Thr Ser His Ile Asp
 -5                  -1   1               5                  10

cag gag tcc tac aac ttc ttt gag aag tac gcc cga ctc gca aac att    3916
Gln Glu Ser Tyr Asn Phe Phe Glu Lys Tyr Ala Arg Leu Ala Asn Ile
                15                  20                  25

gga tat tgt gtt ggt ccc ggc act aag atc ttc aag ccc ttc aac tgt    3964
Gly Tyr Cys Val Gly Pro Gly Thr Lys Ile Phe Lys Pro Phe Asn Cys
            30                  35                  40

ggc ctg caa tgt gcc cac ttc ccc aac gtt gag ctc atc gag gag ttc    4012
Gly Leu Gln Cys Ala His Phe Pro Asn Val Glu Leu Ile Glu Glu Phe
    45                  50                  55
```

```
cac gac ccc cgt ctc atc ttt gat gtt tct ggt tac ctc gct gtt gat    4060
His Asp Pro Arg Leu Ile Phe Asp Val Ser Gly Tyr Leu Ala Val Asp
 60                  65                  70                  75

cat gcc tcc aag cag atc tac ctt gtt att cga gga acc cac tct ctg    4108
His Ala Ser Lys Gln Ile Tyr Leu Val Ile Arg Gly Thr His Ser Leu
                 80                  85                  90

gag gac gtc ata acc gac atc cga atc atg cag gct cct ctg acg aac    4156
Glu Asp Val Ile Thr Asp Ile Arg Ile Met Gln Ala Pro Leu Thr Asn
             95                 100                 105

ttt gat ctt gct gct aac atc tct tct act gct act tgt gat gac tgt    4204
Phe Asp Leu Ala Ala Asn Ile Ser Ser Thr Ala Thr Cys Asp Asp Cys
        110                 115                 120

ctt gtc cac aat ggc ttc atc cag tcc tac aac aac acc tac aat cag    4252
Leu Val His Asn Gly Phe Ile Gln Ser Tyr Asn Asn Thr Tyr Asn Gln
    125                 130                 135

atc ggc ccc aag ctc gac tct gtg att gag cag tat ccc gac tac cag    4300
Ile Gly Pro Lys Leu Asp Ser Val Ile Glu Gln Tyr Pro Asp Tyr Gln
140                 145                 150                 155

att gct gtc acc ggt cac tct ctc gga gga gct gca gcc ctt ctg ttc    4348
Ile Ala Val Thr Gly His Ser Leu Gly Gly Ala Ala Ala Leu Leu Phe
                160                 165                 170

gga atc aac ctc aag gtt aac ggc cac gat ccc ctc gtt gtt act ctt    4396
Gly Ile Asn Leu Lys Val Asn Gly His Asp Pro Leu Val Val Thr Leu
            175                 180                 185

ggt cag ccc att gtc ggt aac gct ggc ttt gct aac tgg gtc gat aaa    4444
Gly Gln Pro Ile Val Gly Asn Ala Gly Phe Ala Asn Trp Val Asp Lys
        190                 195                 200

ctc ttc ttt ggc cag gag aac ccc gat gtc tcc aag gtg tcc aaa gac    4492
Leu Phe Phe Gly Gln Glu Asn Pro Asp Val Ser Lys Val Ser Lys Asp
    205                 210                 215

cga aag ctc tac cga atc acc cac cga gga gat atc gtc cct caa gtg    4540
Arg Lys Leu Tyr Arg Ile Thr His Arg Gly Asp Ile Val Pro Gln Val
220                 225                 230                 235

ccc ttc tgg gac ggt tac cag cac tgc tct ggt gag gtc ttt att gac    4588
Pro Phe Trp Asp Gly Tyr Gln His Cys Ser Gly Glu Val Phe Ile Asp
                240                 245                 250

tgg ccc ctg atc cac cct cct ctc tcc aac gtt gtc atg tgc cag ggc    4636
Trp Pro Leu Ile His Pro Pro Leu Ser Asn Val Val Met Cys Gln Gly
            255                 260                 265

cag agc aat aaa cag tgc tct gcc ggt aac act ctg ctc cag cag gtc    4684
Gln Ser Asn Lys Gln Cys Ser Ala Gly Asn Thr Leu Leu Gln Gln Val
        270                 275                 280

aat gtg att gga aac cat ctg cag tac ttc gtc acc gag ggt gtc tgt    4732
Asn Val Ile Gly Asn His Leu Gln Tyr Phe Val Thr Glu Gly Val Cys
    285                 290                 295
```

```
ggt atc taa gctatttatc actctttaca acttctacct caactatcta        4781
Gly Ile
300

ctttaataaa tgaatatcgt ttattctcta tgattactgt atatgcgttc ctctaagaca 4841

aatcgaaacc agcatgcgat cgaatggcat acaaaagttt cttccgaagt tgatcaatgt 4901

cctgatagtc aggcagcttg agaagattga cacaggtgga ggccgtaggg aaccgatcaa 4961

cctgtctacc agcgttacga atggcaaatg acgggttcaa agccttgaat ccttgcaatg 5021

gtgccttgga tactgatgtc acaaacttaa gaagcagccg cttgtcctct tcctcgaaac 5081

tctcaaacac agtccagagg tcctttatag cttgatctgt atccagatag cctccgtaat 5141

tggtgtgtgt cttcaaatcc cagacgtcca cattggcatg tcctccactg ataagcattt 5201

gaagttcatc tgcgttgaac attgagaccc acgaagggtc aatgagctgg tatagaccgc 5261

ccaagaatgc atctgtctgt gttctgatac tggtgttaag ctt                   5304
```

<210> 2
<211> 334
<212> PRT
<213> Yarrowia lipolytica

<400> 2

```
Met Lys Leu Ser Thr Ile Leu Phe Thr Ala Cys Ala Thr Leu Ala Ala
1               5                   10                  15
Ala Leu Pro Ser Pro Ile Thr Pro Ser Glu Ala Ala Val Leu Gln Lys
            20                  25                  30
Arg Val Tyr Thr Ser Thr Glu Thr Ser His Ile Asp Gln Glu Ser Tyr
        35                  40                  45
Asn Phe Phe Glu Lys Tyr Ala Arg Leu Ala Asn Ile Gly Tyr Cys Val
    50                  55                  60
Gly Pro Gly Thr Lys Ile Phe Lys Pro Phe Asn Cys Gly Leu Gln Cys
65                  70                  75                  80
Ala His Phe Pro Asn Val Glu Leu Ile Glu Glu Phe His Asp Pro Arg
                85                  90                  95
Leu Ile Phe Asp Val Ser Gly Tyr Leu Ala Val Asp His Ala Ser Lys
            100                 105                 110
Gln Ile Tyr Leu Val Ile Arg Gly Thr His Ser Leu Glu Asp Val Ile
        115                 120                 125
Thr Asp Ile Arg Ile Met Gln Ala Pro Leu Thr Asn Phe Asp Leu Ala
    130                 135                 140
Ala Asn Ile Ser Ser Thr Ala Thr Cys Asp Asp Cys Leu Val His Asn
145                 150                 155                 160
Gly Phe Ile Gln Ser Tyr Asn Asn Thr Tyr Asn Gln Ile Gly Pro Lys
                165                 170                 175
Leu Asp Ser Val Ile Glu Gln Tyr Pro Asp Tyr Gln Ile Ala Val Thr
            180                 185                 190
Gly His Ser Leu Gly Gly Ala Ala Ala Leu Leu Phe Gly Ile Asn Leu
        195                 200                 205
Lys Val Asn Gly His Asp Pro Leu Val Val Thr Leu Gly Gln Pro Ile
    210                 215                 220
Val Gly Asn Ala Gly Phe Ala Asn Trp Val Asp Lys Leu Phe Phe Gly
225                 230                 235                 240
Gln Glu Asn Pro Asp Val Ser Lys Val Ser Lys Asp Arg Lys Leu Tyr
                245                 250                 255

Arg Ile Thr His Arg Gly Asp Ile Val Pro Gln Val Pro Phe Trp Asp
            260                 265                 270
Gly Tyr Gln His Cys Ser Gly Glu Val Phe Ile Asp Trp Pro Leu Ile
        275                 280                 285
His Pro Pro Leu Ser Asn Val Val Met Cys Gln Gly Gln Ser Asn Lys
    290                 295                 300
Gln Cys Ser Ala Gly Asn Thr Leu Leu Gln Gln Val Asn Val Ile Gly
305                 310                 315                 320
Asn His Leu Gln Tyr Phe Val Thr Glu Gly Val Cys Gly Ile
                325                 330
```

## Description

La présente invention est relative à la production par génie génétique, de lipases de levures utilisables pour l'obtention de médicaments.

Les aliments ingérés quotidiennement par l'homme sont constitués essentiellement de lipides, de protéines et de sucres. Tous ces constituants subissent, avant leur absorption, une hydrolyse catalysée par les enzymes du tractus digestif. Un déficit en l'une ou l'autre de ces enzymes peut donc entraîner des troubles digestifs, et conduire à une malnutrition importante. C'est par exemple le cas dans certaines situations pathologiques, comme la mucoviscidose ou l'insuffisance pancréatique exocrine, auxquelles est associé un déficit en lipase pancréatique. Il est classiquement proposé pour corriger ce déficit, d'administrer par voie orale des extraits pancréatiques. Toutefois l'efficacité de cette thérapeutique est limitée par le fait que les enzymes contenues dans ces extraits (lipases, amylases et protéases) sont rapidement inactivées par l'acidité du milieu gastrique.

Il a donc été proposé d'utiliser des préparations de lipases résistant aux conditions gastriques, telles que par exemple des préparations de lipase gastrique de mammifères produites par génie génétique, comme celle décrite dans la Demande de Brevet Français publiée sous le numéro 2 699 179, ou des préparations de lipases de microorganismes possédant une activité correcte en milieu acide [ZENTLER-MONRO et al., Pancreas, 7, 311-319 (1992)].

Parmi les lipases de microorganismes actives en milieu acide, on citera en particulier des lipases fongiques, telles que celles de *Candida ernobii,* [YOSHIDA et al., Biochim. Biophys. Acta,; 154, 586-588, (1968)], de *Trichosporon asteroide* [DHARMSTHITI et al., Biotechnol. Appl. Biochem., 26, 111-116 (1997)], de *Rhizopus javanicus* [(UYTTENBROECK et al., Biol. Chem. Hoppe Seyler, 374, 245-254, (1993)], ou de *Yarrowia lipolytica* [HADEBALL, Acta Biotechnol., 2, 159-167 (1991) ; NOVOTNY et al., J. Basic Microbiol., 28, 221-227 (1988)]. Outre leur activité à pH acide, ces lipases présentent les caractéristiques communes d'être résistantes, en présence de leur substrat, à la digestion par les protéases (trypsine, chymotrypsine et pepsine), et d'être résistantes à l'action des sels biliaires (leur activité est conservée en présence de 10 mM de taurocholate de sodium.

Cependant, la production naturelle de lipases par ces microorganismes n'est généralement pas suffisante pour permettre de les préparer à l'échelle industrielle à un coût raisonnable.

Les Inventeurs ont entrepris d'isoler et de cloner le gène d'une lipase fongique active à pH acide, afin de permettre sa surexpression dans des cellules hôtes.

Ils sont ainsi parvenus à cloner le gène codant pour une lipase extracellulaire acidorésistante de *Yarrowia lipolytica* (également dénommée ci-après LIP2 ou LIP2w29a).

L'expression de ce gène dans des cellules hôtes leur a permis d'obtenir cette enzyme sous forme active.

La présente invention a pour objet un fragment d'acide nucléique isolé comprenant une séquence codant pour le précurseur intracellulaire d'une lipase extracellulaire acidorésistante de levure, caractérisé en ce que ledit précurseur intracellulaire de ladite lipase présente, au niveau de la séquence polypeptidique, au moins 30% d'identité, et au moins 39% de similarité avec le précurseur intracellulaire de la lipase extracellulaire de *Yarrowia lipolytica,* défini par la séquence SEQ ID NO:2.

Par : « fragment d'acide nucléique codant pour une lipase extracellulaire » on entend tout fragment d'acide nucléique comprenant au moins une séquence codant pour la forme mature de ladite lipase ; il peut comprendre en outre tout ou partie des séquences codant pour les portions « pré » et « pro » de son précurseur intracellulaire.

Des molécules d'acide nucléique conformes à l'invention sont représentées, par exemple, par la séquence nucléotidique SEQ ID NO:1 de la liste de séquences en annexe.

Dans la séquence SEQ ID NO:1, les nucléotides 3737 à 4741 correspondent à la séquence codant pour le précurseur intracellulaire de la lipase extracellulaire de *Y. lipolytica,* et les nucléotides 3836 à 4741 codent pour l'enzyme mature sécrétée par les cellules.

Le polypeptide de séquence SEQ ID NO:2 comprend au total 335 acides aminés, et la masse moléculaire théorique déduite de sa séquence est de 36782 daltons. La protéine mature comprend 301 acides aminés.

Selon un mode de réalisation avantageux du fragment d'acide nucléique, il comprend au moins une séquence codant pour un polypeptide comprenant les acides aminés 34 à 334 du polypeptide défini par la séquence SEQ ID NO: 2. Dans ce cas, ledit fragment comprend avantageusement la séquence correspondant aux nucléotides 3836-4738 de la SEQ ID NO:1.

Selon une disposition avantageuse de ce mode de réalisation, ledit fragment d'acide nucléique comprend en outre une séquence codant pour un polypeptide permettant la sécrétion de ladite lipase. Dans ce cas, ledit fragment comprend avantageusement une séquence codant pour les acides aminés 1 à 33 du polypeptide défini par la séquence SEQ ID NO:2.

Les résultats de la comparaison de la de la séquence SEQ ID NO:2 correspondant au précurseur intracellulaire, avec des séquences de la banque de données SWISSPROT, en utilisant le programme GCG-gap [version 9.1-UNIX (sept.97).] et la matrice BLOSUM 62, (GENETICS COMPUTER GROUP, Université du Wisconsin), sont illustrés dans le tableau I ci-dessous.

TABLEAU I

| N° SWISS-PROT | ORGANISME | *Y. lipolytica* | |
|---|---|---|---|
| | | %identité | %similarité |
| | *Yarrowia. lipolytica* | 100 | 100 |
| | *Candida. ernobii* | 30,3 | 39,2 |
| P47145 | *Saccharomyces* cerevisiae[a] | 28,6 | 38,2 |
| P25234 | *Penicillium camenbertii* | 26,8 | 33,6 |
| P21811 | *Rhizopus delemar* | 31,5 | 38,6 |
| P19515 | *Rhizomucor miehei* | 27,4 | 34,1 |
| JX0343 | *Fusarium heterosporum* | 29 | 36 |
| a : lipase hypothétique | | | |

L'invention englobe également des cassettes d'expression d'une lipase extracellulaire acidorésistante de levure, comprenant :

- un fragment d'acide nucléique conforme à l'invention ;
- des séquences permettant de réguler l'expression de ladite lipase dans une cellule-hôte.

Le fragment d'ADN conforme à l'invention comprend la séquence codant pour la forme mature de la lipase acidorésistante, seule ou associée à des séquences codant pour des signaux de sécrétion. Ces signaux de sécrétion peuvent être soit ceux naturellement associés à ladite lipase, soit des signaux hétérologues.

Des séquences permettant la régulation de l'expression sont en particulier des séquences de type promoteur et terminateur de transcription, fonctionnelles dans la cellule-hôte où l'on souhaite exprimer la lipase.

Des séquences de régulation appropriées peuvent être choisies en fonction de la cellule-hôte, des conditions (par exemple inductible ou constitutive) et du niveau d'expression souhaités.

On peut utiliser des séquences de régulation naturellement associées à celle codant pour la lipase, et/ou des séquences hétérologues. Des séquences provenant de différents gènes peuvent être utilisées, associées dans des combinaisons différentes.

On peut ainsi, par exemple, construire une cassette exprimant un précurseur intracellulaire, dans lequel la forme mature d'une lipase acidorésistante codée par un fragment d'acide nucléique conforme à l'invention est associée avec ses propres signaux de sécrétion, ou avec des signaux de sécrétion d'origine hétérologue ; la séquence codant pour ce précurseur peut être placée sous contrôle transcriptionnel de ses propres promoteur et terminateur, ou bien associée à un promoteur hétérologue et/ou à un terminateur hétérologue.

Il est ainsi possible d'utiliser dans le cadre d'une cassette d'expression conforme à l'invention, le promoteur, et/ou le terminateur de transcription du gène de la lipase extracellulaire de *Y. lipolytica,* ainsi que la séquence codant pour tout ou partie de la région pré-pro de la lipase extracellulaire de *Y. lipolytica.*

D'autre part, la séquence codant pour la forme mature de la lipase de *Y. lipolytica* codée par un fragment d'acide nucléique conforme à l'invention, peut être associée avec un promoteur et/ou un terminateur et/ou des signaux de sécrétion hétérologues. Par exemple, pour l'expression dans une levure de la lipase extracellulaire de *Y. lipolytica,* on peut utiliser la séquence signal de cette lipase, et le promoteur et/ou le terminateur du gène LIP2, ou bien d'autres promoteurs et/ou d'autres terminateurs, tel que par exemple le promoteur *ACO2* de *Y. lipolytica* [LE CLAINCHE, Thèse de doctorat de l'Institut National Agronomique Paris Grignon, soutenue le 2 juillet 1997] qui, comme celui de la lipase LIP2, est inductible par les triglycérides et les acides gras.

Pour exprimer une lipase codée par un fragment d'acide nucléique conforme à l'invention dans d'autres cellules hôtes, on associera la séquence codant pour la protéine mature à des séquences de contrôle de la transcription et à des signaux de sécrétion fonctionnels dans cette cellule hôte.

La présente invention englobe également les vecteurs recombinants susceptibles d'être obtenus par l'insertion d'au moins un fragment d'acide nucléique conforme à l'invention, et avantageusement d'au moins une cassette d'expression conforme à l'invention, dans un vecteur hôte.

On choisira de préférence comme vecteurs hôtes des vecteurs permettant la réplication en plusieurs copies de l'information génétique introduite dans la cellule hôte, et avantageusement, son maintien sous forme stable. Il peut s'agir de vecteurs extra-chromosomiques, tels que des plasmides multicopie stables, ou des vecteurs épisomaux ; il peut aussi s'agir de vecteurs intégratifs.

A titre d'exemple de vecteurs hôtes utilisables pour le clonage et l'expression d'une lipase extracellulaire acidorésistante codée par un fragment d'acide nucléique conforme à l'invention on citera par exemple les vecteurs décrits dans la Demande PCT WO 00/12729 au nom de l'INRA.

Une souche d'E. coli hébergeant l'un de ces vecteurs dénommé JMP10, qui comprend un insert correspondant à la région 1715-4806 de la séquence SEQ ID NO: 1, contenant le promoteur, la portion codante et le terminateur de transcription de la lipase de *Y. lipolytica,* a été déposée à la Collection Nationale de Cultures de Microorganismes (CNCM), INSTITUT PASTEUR, 25, rue du Docteur Roux, 75724 Paris Cédex 15, France, le 12 mai 1998, sous le numéro I-2013.

La présente invention a également pour objet des cellules eucaryotes ou procaryotes transformées par au moins un fragment d'acide nucléique conforme à l'invention.

Des cellules transformées conformes à l'invention peuvent par exemple être obtenues à partir de cellules bactériennes, de cellules de levure ou de champignons, ou de cellules animales (par exemple des cellules de mammifères ou d'insectes).

Les Inventeurs ont notamment obtenu, à partir de cellules de *Y. lipolytica* transformées par un fragment d'acide nucléique conforme à l'invention, une souche surproductrice de ladite lipase.

Un autre objet de la présente invention concerne donc cette souche de *Y. lipolytica,* qui a été déposée le 24 août 1999 conformément au traité de Budapest auprès de la Collection Nationale de Cultures de Microorganismes (CNCM), INSTITUT PASTEUR, 25 rue du Docteur Roux, 75724 PARIS CEDEX 15, FRANCE, sous le numéro 1-2294.

L'invention concerne également un procédé de production de lipase recombinante, lequel procédé est caractérisé en ce qu'il comprend au moins une étape au cours de laquelle l'on procède à la mise en culture de cellules transformées conformes à l'invention, et une étape au cours de laquelle on recueille la lipase recombinante produite par lesdites cellules.

Selon un mode de mise en oeuvre préféré du procédé conforme à l'invention, lesdites cellules transformées sont des cellules de la souche I-2294.

Cette lipase recombinante peut être obtenue soit après lyse cellulaire, soit, avantageusement, à partir du milieu de culture dans lequel elle est sécrétée par les cellules, par des techniques classiques, connues en elles-mêmes, par exemple par précipitation fractionnée suivie d'une ou plusieurs étapes de chromatographie.

La présente invention sera mieux comprise à l'aide du complément de description qui va suivre, qui se réfère à des exemples décrivant le clonage du gène codant pour la lipase extracellulaire de *Y. lipolytica,* et l'obtention de souches de levure transformée exprimant ce gène.

Il va de soi toutefois que ces exemples sont donnés uniquement à titre d'illustration de l'objet de l'invention dont ils ne constituent en aucune manière une limitation.

## EXEMPLE 1 : ISOLATION ET CARACTERISATION DU GENE DE LA LIPASE DE *YARROWIA LIPOLYTICA.*

Isolation de l'ADNc :

Une banque d'ADNc de *Yarrowia lipolytica* a été criblée à l'aide de sondes d'acide nucléique dérivées de la séquence codant pour la lipase de *Candida ernobii.*

A partir des clones positifs, le gène de la lipase de *Yarrowia lipolytica* (LIP2W29a) a été amplifié par PCR à l'aide du couple d'oligonucléotides convergents Lip ATG/Lip STOP :

Lip ATG :
5'
CCCGGGCCGCAGTGGCCACAATGAAGCTTTCCACCATCCTTTTCACAGCC
3'

Lip STOP :
5'
GTTGGCGGCCGCGAATTCGGATCCTAAATAAACGATATTCATTTATTAAAGTAGATAGTTGAGG
3'

et cloné dans le plasmide pBLUESCRIPT KS+ (STRATAGENE, La Jolla) au site EcoRV.

Le plasmide recombinant obtenu est dénommé pKS-LIP2W29a. Il contient un insert de 1118 pb (coordonnées 3737 à 4806 de la séquence SEQ ID N0: 1) contenant les sites de restriction SmaI et SfiI, suivis d'une séquence consensus de l'environnement de l'ATG (CACAATG), de la séquence du cadre de lecture de la prolipase, de la séquence terminatrice jusqu'au site de polyadénylation, et des sites de restriction BamHI, EcoRI et NotI.

Isolation de l'ADN génomique :

Les six pools d'une banque d'ADN génomique de *Y lipolytica* [XUAN et al., C. Curr. Genet., 14, 15-21, (1988) ] ont été testés à l'aide du couple d'oligonucléotides convergents Ylip04 et Ylip05.
Ylip04 5' GGCACTAAGATCTTCAAGCCC 3'
Ylip05 5' GAAGCCATTGTGGACAAGACAG 3'

Une amplification a été obtenue pour le pool 2. Les colonies bactériennes de ce pool ont été testées par hybridation avec le gène LIP2W29a, obtenu comme décrit en 1) ci-dessus, comme sonde. Ce criblage a permis d'obtenir le plasmide pINA-LIP2, qui contient un fragment génomique d'environ 6,5 kb (Figure 1-C). Ce fragment a été digéré par différentes enzymes de restriction. Les fragments EcoRV (E1), PstI (P1, P2, P3 et P4) et BglII (B1) ainsi obtenus ont été clonés dans le plasmide pBLUESCRIPT KS+ aux sites EcoRV, PstI et BamHI, respectivement. L'analyse du plasmide pINA-LIP2 a révélé qu'il contient les régions promotrices et une partie de la séquence codant pour la lipase.

Afin d'obtenir la fin du gène de la lipase et la région terminatrice une étape de PCR inverse a été réalisée. L'hybridation de l'ADN génomique coupé par les enzymes de restriction HindIII, PstI et SphI, avec le gène *LIP2W29a* a révélé des fragments de 1,4 kb, 1,66 kb et 3,7 kb, respectivement. Le fragment HindIII de 1,4 kb d'ADN génomique a été amplifié : l'ADN génomique a été digéré par HindIII, ligué et amplifié par PCR à l'aide des oligonucléotides divergents Ylip03 et Ylip04. Ylip03 :
5' TATCCAATGTTTGCGAGTCGGG 3'

Le fragment obtenu (Figure 1-D) a été cloné dans le plasmide pBLUESCRIPT KS+ au site EcoRV. Le plasmide recombinant, dénommé pKS-PCR-H a été séquencé.

Les séquences d'une partie de l'insert de pINA-LIP2, du plasmide pKS-LIP2W29a et du plasmide pKS-PCR-H ont été assemblées, pour obtenir un segment EcoRV-HindIII de 5304 pb (Figure 1-E) dont la séquence figure en annexe sous le numéro SEQ ID N0 :1.

La Figure 1 schématise les différentes étapes, décrites ci-dessus, du clonage du gène de la lipase extracellulaire de *Yarrowia lipolytica* (LIP2w29a).

Légende de la Figure 1 :

A : Carte de restriction génomique partielle du locus de LIP2w29a. Les symboles représentent EcoRV (EV), PstI (P), BglII (Bg) et HindIII (H).
La localisation de l'ORF de la LIP2w29a est schématisée par un bloc noir.
B : Localisation de la séquence de l'ADNc et représentation schématique des oligonucléotides Lip ATG et Lip STOP utilisés pour amplifier le gène codant pour la lipase. Le plasmide recombinant est appelé pKS-LIP2W29a. Localisation des oligonucléotides Ylip04 et Ylip05 utilisés pour tester la banque de Xuan (Xuan et al, 1988).
C : Représentation schématique de l'insert de 6,5 kb contenu dans le plasmide pINA-LIP2 et localisation des sous clones EcoRV (E1), PstI (P1, P2, P3 et P4) et BglII (B1) utilisés pour séquencer la région 5' du gène de la LIP2W29a.
D : Séquençage du fragment d'ADN génomique HindIII de 1,4 kb. L'ADN génomique a été digéré par HindIII, ligué et amplifié par PCR avec le couple d'oligonucléotides divergent Ylip03 et Ylip04. Le fragment de PCR a été cloné et le plasmide recombinant contenant ledit fragment appelé pKS-PCR-H.
E : Séquence du locus YL-LIP2. Les séquences du plasmide pKS-LIP2W29a (B), des sous-clones de pINA-LIP2 (C), et du plasmide pKS-PCR-H (D) ont été assemblées. La séquence finale représente une région EcoRV-HindIII de 5304 pb.

L'analyse de la séquence nucléotidique de la région EcoRV-HindIII de 5304 pb a permis de mettre en évidence la présence d'une phase ouverte de lecture codant pour un polypeptide de 335 acides aminés. La séquence de ce polypeptide est représentée dans la liste de séquences en annexe sous le numéro SEQ ID NO :2.

**Caractéristiques de la lipase**

Séquence signal :

La région pré-pro est située entre les positions -33 et-1 de la séquence peptidique. Elle comprend une séquence signal de 13 acides aminés situés entre les positions -33 et -19 de la séquence peptidique, 4 dipeptides X-Ala ou X-Pro, substrats de la diaminopeptidase, une région pro de 12 acides aminés située entre les positions -12 et -1 de la séquence peptidique et dont les deux derniers acides aminés forment un site KR (Lys-Arg) substrat d'une endoprotéase codée par le gène XPR6 (homologue de Kex2 chez S. *cerevisiae)* .

Sites potentiels de N-glycosylation :

Il existe deux sites potentiels de N-glycosylation (Asparagine-X-Ser/Thr) qui sont localisés au niveau des acides aminés 113 et 134.

Site catalytique :

On retrouve les trois acides aminés vraisemblablement impliqués dans la composition du site catalytique de l'enzyme aux positions respectives suivantes: 162 pour le résidu sérine compris dans la séquence consensus GxSxG commune à toutes les lipases, 230 pour le résidu acide aspartique et en 289 pour le résidu histidine.

L'analyse par transfert de Southern montre que ce gène est présent en une seule copie dans le génome de *Yarrowia lipolytica.*

**EXEMPLE 2 : CLONAGE DU GENE DE LA LIPASE LIP2 DE *YARROWIA LIPOLYTICA* DANS DES VECTEURS INTEGRATIFS ET EXPRESSION CHEZ *YARROWIA LIPOLYTICA :***

**Construction des vecteurs comprenant le gène codant pour la lipase de *Yarrowia lipolytica.***

Le gène codant pour la lipase LIP2 de *Yarrowia lipolytica* a été cloné dans le vecteur intégratif JMP3. Ce vecteur hôte est décrit dans la Demande Française au nom de l'INRA, intitulée : « PROCEDE DE TRANSFORMATION NON-HOMOLOGUE DE YARROWIA LIPOLYTICA » et déposée le même jour que la présente Demande.

Il s'agit d'un vecteur d'autoclonage qui s'intègre dans le génome après coupure par l'enzyme de restriction NotI. Cette restriction permet l'élimination de la partie bactérienne (origine de réplication de E. coli et du marqueur de résistance KanR).

Ils sont dérivés du vecteur pINA1067 (également dénommé pINA970) décrit par BARTH et GAILLARDIN [The dimorphic fungus Yarrowia lipolytica. In: Non conventional yeasts in biotechnology (Wolf K. Ed.). Springer-Verlag, Berlin pp 313-388, (1996)], dans lequel a été introduit un fragment NotI comprenant une origine de réplication pour E. coli et un marqueur de résistance à la kanamycine, et d'où a été excisé un fragment HindIII-EcoRI (contenant des séquences du plasmide pBR322, un marqueur de résistance à la tétracycline et le promoteur et le terminateur XPR2) qui a été remplacé par un adaptateur contenant les sites de restriction HindIII, ClaI, MluI, HpaI, BamHI et EcoRI.

Construction des vecteurs d'amplification JMP6 et JMP10.

Les vecteurs d'amplification JMP6 et JMP10 ont été construits par ligation trois voies en utilisant des fragments purifiés après électrophorèse sur gel d'agarose.

*Construction* de *JMP6 :*

Le promoteur du gène de l'acyl CoA oxidase *ACO2* de *Y. lipolytica* a été amplifié par PCR avec les oligonucléotides Aco2P1 et Aco2P2 qui contiennent un site ClaI et HindIII, respectivement. Le fragment de PCR de 2168 pb a été cloné au site EcoRV du vecteur BLUESCRIPT KS+. Le plasmide résultant est appelé KS-ACO2prom. Ce vecteur a été digéré par ClaI et partiellement par HindIII pour obtenir un fragment de 2155pb contenant le promoteur complet.

Le gène de la lipase est contenu dans un fragment HindIII-EcoRI de 1134pb isolé du plasmide KS+-LIP2w29a.

Les deux fragments ont été purifiés et insérés aux sites ClaI-EcoRI du vecteur JMP3. Le plasmide résultant est appelé JMP6.

Ce plasmide est représenté sur la Figure 2. *Construction* de *JMP10 :*

Le promoteur du gène de la lipase LIP2 de *Y. lipolytica* est porté par un fragment NdeI*-HindIII de 2030 pb du plasmide KS+-LIP2prom. Ce plasmide a été digéré par NdeI, traité à la T4 DNA polymérase pour rendre le site NdeI franc (NdeI*). Après inactivation de l'enzyme NdeI et de la polymérase (20 min. 70°C), l'ADN a été coupé par HindIII. Le fragment NdeI*-HindIII de 2030 pb a été ligué avec le fragment HindIII-EcoRI de 1134 pb portant le gène de la lipase et son terminateur et inséré aux sites HpaI-EcoRI du vecteur JMP3.

Le plasmide résultant appelé JMP10, est représenté sur la Figure 3. Ce plasmide a été déposé à la Collection Nationale de Cultures de Microorganismes (CNCM), INSTITUT PASTEUR, 25, Rue du Docteur Roux, 75724 Paris Cédex 15, France, le 12 mai 1998, sous le numéro I-2013.

Les plasmides JMP6 et JMP10 ont été introduits par transformation à l'acétate de lithium, après linéarisation par NotI, E150, (comprenant des sites zéta), soit dans la souche PO1d (dépourvue de transposons et de sites zéta). Ces deux souches de *Y. lipolytica* possèdent une délétion du gène *URA3.*

On obtient typiquement 20 à 50 transformants/μg de DNA avec la souche PO1d et 100 à 200 transformants/μg de DNA avec la souche E150.

Les transformants sont désignés ci-après selon la nomenclature suivante : nom de la souche - numéro du plasmide - numéro du transformant. Exemple ; PO1d-6-15 souche PO1d plasmide JMP6 transformant numéro 15.

L'intégration du gène de la lipase dans le génome des transformants est vérifié par transfert de SOUTHERN, et hybridation avec des sondes dérivées des séquences ZETA, de la séquence du promoteur ACO2, et de la séquence de la lipase.

**Production de lipase dans le milieu de culture par les souches transformées de Yarrowia lipolytica**

Les différentes souches sont mises en culture à 28°C sous agitation (200 rpm) dans un erlen à ailettes de 250 ml contenant 40ml de milieu (Extrait de levure 10 g/l ; Bactopeptone 20 g/l ; tampon phosphate de sodium 50 mM, pH 6,8) avec ou sans inducteur (par exemple 0.5% d'oléate). L'ensemencement initial se fait à une densité optique de 0,1 unité par ml à 600 nm.

Différents prélèvements sont opérés au cours du temps et les tests d'activité lipase sont réalisés sur les surnageants par titrimétrie, selon le protocole suivant :

Les mélanges réactionnels (5 ml d'émulsion d'huile d'olive (SIGMA, France, ref. 800-1) ; 2 ml de tampon $Na_2HPO_4/KH_2PO_4$ 50 mM, pH 6,8 ; enzyme à différentes concentrations) sont incubés 20 min à 37°C sous agitation orbitale (250 rpm) dans des tubes en plastique de 20 ml. La réaction est arrêtée par addition de 4 ml de solution d'un mélange 1/1 (v/v) éthanol/acétone, contenant 0,09% de thymolphtaléine (w/v). Le contenu des tubes est ensuite titré avec de la soude 50 mM (soude O,OSN (TITRISOL, MERCK)) jusqu'à l'apparition du virage coloré bleu de la thymolphtaléine. L'activité enzymatique présente dans les tubes est calculée comme suit :

$$\text{Activité (U/ml)} = \frac{(V-Vo) \times M \times D}{(T \times E)}$$

Vo = volume de soude mis dans l'échantillon témoin pour le dosage (ml)
V = volume de soude mis dans l'échantillon (ml)
T = temps de la réaction (min)
M = molarité de la soude utilisée pour le dosage (mM)
D = dilution effectuée sur l'échantillon dosé
E = volume de l'échantillon utilisé pour le dosage (ml)

Une unité lipase correspond à la quantité d'enzyme capable de catalyser la formation d'1 μmole d'acide gras/min dans les conditions opératoires décrites ci-dessus.

Les résultats obtenus avec la souche de référence E150 et les souches modifiées E150-6-2 et E150-10-4, sont illustrés dans le tableau II ci-dessous :

TABLEAU II

| | activité lipase (unités/ml de surnageant) | | | | | |
|---|---|---|---|---|---|---|
| | milieu sans inducteur | | | milieu avec inducteur | | |
| temps de culture (heures) | E150 | E150-6-2 | E150-10-4 | E150 | E150-6-2 | E150-10-4 |
| 0 | 0 | 0 | 0 | 0 | 0 | 0 |
| 4 | 0 | 0 | | 0 | 0 | |
| 7,5 | 0 | 4 | 0 | 4 | 8 | 12 |
| 23 | 8 | 22 | 29 | 12 | 229 | 92 |
| 32 | 4 | 46 | | 13 | 312 | |

(suite)

| temps de culture (heures) | E150 | E150-6-2 | E150-10-4 | E150 | E150-6-2 | E150-10-4 |
|---|---|---|---|---|---|---|
| 46 | 0 | 21 | 58 | 17 | 280 | 92 |
| 70 | 0 | 12 | | 13 | 262 | |

Les résultats obtenus avec la souche de référence PO1d et le clone transformé PO1d-6-17 sont illustrés par le tableau III ci-dessous :

TABLEAU III

| | activité lipase (U/ml) | |
|---|---|---|
| | milieu avec inducteur | |
| temps de culture (heures) | PO1d | PO1d-6-17 |
| 0 | 0 | 0 |
| 8 | 0 | 15 |
| 10 | 0 | 40 |
| 13 | 0 | 160 |
| 15 | 14 | 221 |
| 24 | 42 | 229 |
| 36 | 40 | 187 |

**EXEMPLE 3 : PRODUCTION DE LIPASE EXTRACELLULAIRE PAR LE CLONE MS4 DE *YARROWIA LIPOLYTICA***

A partir de cultures de cellules PO1d de *Yarrowia lipolytica* transformées comme décrit dans l'exemple 2, les Inventeurs sont parvenus à sélectionner un clone produisant une quantité de lipase beaucoup plus importante que les autres clones transformés, sans que cette surproduction nuise à ses capacités de croissance.

Ce clone dénommé ci-après MS4, contient 10 copies de la cassette d'expression ; il a été déposé le 24 août 1999 auprès de la Collection Nationale de Cultures de Microorganismes (CNCM), INSTITUT PASTEUR, 25 rue du Docteur Roux, 75724 PARIS CEDEX 15, FRANCE, sous le numéro I-2294.

La production de lipase par ce clone est déterminée comme décrit ci-après.

**A/ Production de lipase extracellulaire par le clone MS4 en Erlenmeyer**

Conditions expérimentales

Toutes les cultures sont réalisées à 28°C sous agitation (150 rpm) dans des fioles à ailettes bouchées de 250 ml (CML, France) contenant 50 ml de milieu de culture (eau désionisée ; extrait de levure 10g/l ; bactotryptone N1 20 g/l (ORGANOTECHNIE) ; tampon $Na_2HPO_4/KH_2PO_4$ 50 mM, pH 6,8 ; glucose 10 g/l (SIGMA) ; huile d'olive ou acide oléique (MERCK) à différentes concentrations).

Les ensemencements initiaux des cultures sont réalisés de façon à obtenir une densité optique à 600 nm ($DO_{600nm}$) de 0,25 pour 1 ml (soit environ $2,5.10^7$ cellules/ml).

Plusieurs prélèvements sont effectués au cours de la réaction. Pour chaque prélèvement, la biomasse et le surnageant sont séparés par centrifugation et leur $DO_{600nm}$ est mesurée pour 1 ml. L'activité lipase est dosée dans le surnageant par titrimétrie, comme indiqué à l'Exemple 2 ci-dessus tandis que la croissance cellulaire est mesurée à partir de la biomasse.

Résultats

Sur la Figure 4 sont représentées :

- la croissance cellulaire du clone MS4 (O) évaluée par la mesure de l'absorbance à 600nm $A_{600nm}$ en fonction du temps.

- l'activité lipase (●) en fonction du temps.

Ces résultats montrent que dans ces conditions de culture, l'activité lipase augmente dans le surnageant pour atteindre un maximum à 96 heures ; le clone MS4 permet la production d'environ 0,5 g/l de lipase, soit 12000 U/ml (Figure 4), dans le surnageant après 100 heures de culture. Ceci représente 200 fois plus de lipase que la souche PO1d initiale.

L'analyse en gel SDS-PAGE de la cinétique de production de la lipase dans le surnageant de culture montre une accumulation constante de la lipase sans dégradation apparente.

Les inventeurs ont en outre testé l'influence des conditions de culture et de la composition du milieu sur la production de lipase.

Ils ont ainsi constaté que l'utilisation de bactotryptone favorisait fortement l'activité lipase. En effet, lorsque ce constituant est remplacé par une bactopeptone, l'activité observée est environ 3 fois moindre.

Il a également été observé que la présence d'un acide gras ou d'un triglycéride était indispensable pour l'induction de la production de la lipase. La présence de 5% d'huile d'olive dans la culture permet d'obtenir les titres maximaux de lipase, l'ajout de 10% d'huile d'olive n'augmentant pas significativement les titres. L'acide oléique est également un excellent inducteur lorsqu'il est utilisé à 10%.

**B/ Production de lipase extracellulaire en fermenteur**

- Conditions expérimentales

*- Préparation des ampoules d'ensemencement*

Le clone MS4 est étalé à forte densité sur milieu minimum agar (YNB ww DIFCO 1,7 g/l ; glutamate 0,5 g/l ; glucose 10 g/l ; PASTAGAR B 12 g/l) et est mis à incuber 48 h à 28°C. Les cellules sont récupérées avec 5 ml de YPD et sont utilisées pour ensemencer 200 ml de milieu YPD. Elles sont ensuite cultivées jusqu'à une $A_{600nm}$ de 10 (soit $10^8$ cellules/ml). Les levures sont reprises après centrifugation dans 50 ml de YEA glycérol (extrait de levure 5 g/l ; glucose 10 g/l ; glycérol 20%) froid et conservées en ampoules de 1 ml à - 80°C.

*- Préculture*

Deux précultures sont réalisées pendant 16 heures à 28°C, sous agitation orbitale (150 rpm), dans des fioles à ailettes de 11 contenant 200 ml de milieu YPD (extrait de levure (LIFE TECHNOLOGIES France) 10 g/l ; bactopeptone (DIFCO, France) 10 g/l ; glucose anhydre (SIGMA, France) 10 g/l).

Chaque préculture est ensemencée avec une ampoule de 1 ml du clone MS4 (soit $4.10^8$ cellules).

*- Fermentation*

*•Milieu de fermentation en lots* :

eau désionisée, extrait de levure (ORGANOTECHNIE, France) 10 g/l ; Bactotryptone (ORGANOTECHNIE, France) 20 g/l ; tampon $Na_2HPO_4/KH_2PO_4$ 50 mM, pH 6,8 ; huile d'olive 5% (huile d'olive ROUX, France) ; 3,5 ml d'antimousse (RHODORSIL 426 R, PROLABO), ajouté en une seule fois dès le début de la fermentation.

Le tampon phosphate et l'huile d'olive sont autoclavés séparément.

*• Milieu de fermentation en continu* :

extrait de levure (ORGANOTECHNIE, France) 5 g/l ; bactotryptone (ORGANOTECHNIE, France) 10 g/l ; tampon $Na_2HPO_4/KH_2PO_4$ 50 mM, pH 6,8.

Conditions de la fermentation :

L'ensemencement initial du fermenteur est effectué à une $DO_{600nm}$ de 1 pour 1 ml.

La fermentation est réalisée à 27°C et à pH 6 (pH régulé par de la soude 2,5M), dans un fermenteur de 5 1 contenant 3,5 1 de milieu (voir ci-dessus), avec une aération de 1 vvm, une $pO_2$ fixée à la valeur minimale de 5%, avec une vitesse d'agitation de départ de 300 rpm, pouvant monter à 1000 rpm par incrément de 5 rpm pour conserver la $pO_2$ minimale).

La fermentation dure 24 h en milieu de fermentation en lots et jusqu'à 3 jours en milieu de fermentation en continu.

Résultats :

• *Fermentation en lots*

Une production maximale de l'activité lipase est obtenue après 24 h (4000 U/ml). L'activité se maintient ensuite à un plateau pendant quelques heures avant de commencer à décroître. Cette décroissance semble liée à la disparition de l'huile d'olive, substrat de la lipase, dans le milieu.

• *Fermentation en continu*

Une production de lipase sur 2 jours avec un débit de 200 ml/h a pu être obtenue et un taux de production d'enzyme aux environs de 80 U/ml/h a été obtenu.

## Revendications

1. Fragment d'acide nucléique isolé, comprenant une séquence codant pour le précurseur intracellulaire d'une lipase extracellulaire acidorésistante de levure, **caractérisé en ce que** ledit précurseur intracellulaire de ladite lipase présente au niveau de la séquence polypeptidique, au moins 30% d'identité, et au moins 39% de similarité avec le précurseur intracellulaire de la lipase extracellulaire de *Yarrowia lipolytica,* défini par la séquence SEQ ID NO:2.

2. Fragment d'acide nucléique isolé selon la revendication 1, **caractérisé en ce qu'**il comprend au moins une séquence nucléotidique codant pour un polypeptide comprenant les acides aminés 34 à 334 du polypeptide défini par la séquence SEQ ID NO:2.

3. Fragment d'acide nucléique isolé selon la revendication 2, **caractérisé en ce qu'**il comprend la séquence correspondant aux nucléotides 3836 à 4738 de la séquence SEQ ID NO: 1.

4. Fragment d'acide nucléique isolé selon l'une quelconque des revendications 1 à 3, **caractérisé en ce qu'**il comprend en outre une séquence codant pour un polypeptide permettant la sécrétion de ladite lipase.

5. Fragment d'acide nucléique isolé selon la revendication 4, **caractérisé en ce qu'**il comprend une séquence codant pour les acides aminés 1 à 33 du polypeptide défini par la séquence SEQ ID NO:2.

6. Fragment d'acide nucléique isolé selon la revendication 5, **caractérisé en ce qu'**il comprend la séquence correspondant aux nucléotides 3737 à 3835 de la séquence SEQ ID NO:1.

7. Cassette d'expression d'une lipase extracellulaire acidorésistante de levure, **caractérisée en ce qu'**elle comprend :

   - un fragment d'acide nucléique selon l'une quelconque des revendications 1 à 6 ;
   - des séquences permettant de réguler l'expression de ladite lipase dans une cellule-hôte.

8. Vecteur recombinant comprenant au moins un fragment d'acide nucléique selon l'une quelconque des revendications 1 à 6.

9. Vecteur recombinant selon la revendication 8, comprenant au moins une cassette d'expression selon la revendication 7.

10. Cellule eucaryote ou procaryote transformée par au moins un fragment d'acide nucléique selon l'une quelconque des revendications 1 à 6.

11. Souche de cellule transformée selon la revendication 10 **caractérisée en ce qu'**il s'agit du clone de *Yarrowia lipolytica* dénommé MS4, déposé auprès de la Collection Nationale de Culture de Microorganismes (C.N.C.M.) le 24 août 1999 sous le numéro 1-2294.

12. Procédé de production d'une lipase acidorésistante, **caractérisé en ce qu'**il comprend au moins une étape au cours de laquelle l'on procède à la mise en culture de cellules transformées selon l'une quelconque des revendications 10 ou 11, et une étape au cours de laquelle on recueille la lipase recombinante produite par lesdites cellules.

## Claims

1. Isolated nucleic acid fragment comprising a sequence coding for the intracellular precursor of an acid-resistant extracellular lipase of a yeast, **characterized in that**, in the polypeptide sequence, said intracellular precursor of said lipase has at least 30% identity and at least 39% similarity with the intracellular precursor of the extracellular lipase of *Yarrowia lipolytica,* defined by the sequence SEQ ID NO:2.

2. Isolated nucleic acid fragment according to Claim 1, **characterized in that** it comprises at least one nucleotide sequence coding for a polypeptide comprising amino acids 34 to 334 of the polypeptide defined by the sequence SEQ ID NO:2.

3. Isolated nucleic acid fragment according to Claim 2, **characterized in that** it comprises the sequence corresponding to nucleotides 3836 to 4738 of the sequence SEQ ID NO:1.

4. Isolated nucleic acid fragment according to any one of Claims 1 to 3, **characterized in that** it also comprises a sequence coding for a polypeptide that allows the secretion of said lipase.

5. Isolated nucleic acid fragment according to Claim 4, **characterized in that** it comprises a sequence coding for amino acids 1 to 33 of the polypeptide defined by the sequence SEQ ID NO:2.

**6.** Isolated nucleic acid fragment according to Claim 5, **characterized in that** it comprises the sequence corresponding to nucleotides 3737 to 3835 of the sequence SEQ ID NO:1.

**7.** Expression cassette for an acid-resistant extracellular lipase of a yeast, **characterized in that** it comprises:

- a nucleic acid fragment according to any one of Claims 1 to 6; and
- sequences for regulating the expression of said lipase in a host cell.

**8.** Recombinant vector comprising at least one nucleic acid fragment according to any one of Claims 1 to 6.

**9.** Recombinant vector according to Claim 8 comprising at least one expression cassette according to Claim 7.

**10.** Eukaryotic or prokaryotic cell transformed by at least one nucleic acid fragment according to any one of Claims 1 to 6.

**11.** Transformed cellular strain according to Claim 10, **characterized in that** it is the *Yarrowia lipolytica* clone called MS4 deposited in the Collection Nationale de Culture de Microorganismes (C.N.C.M.) on 24 August 1999 under the number I-2294.

**12.** Process for the production of an acid-resistant lipase, **characterized in that** it comprises at least one step in which transformed cells according to Claim 10 or 11 are cultured, and at least one step in which the recombinant lipase produced by said cells is collected.

**Patentansprüche**

**1.** Isoliertes Nukleinsäurefragment, umfassend eine Sequenz, die für den intrazellulären Vorläufer einer säureresistenten extrazellulären Lipase aus Hefe codiert, **dadurch gekennzeichnet, dass** der intrazelluläre Vorläufer der Lipase auf Polypeptidebene wenigstens 30 % Identität und wenigstens 39 % Ähnlichkeit mit dem durch die Sequenz SEQ ID NO:2 definierten intrazellulären Vorläufer der extrazellulären Lipase von *Yarrowia lipolytica* aufweist.

**2.** Isoliertes Nukleinsäurefragment nach Anspruch 1, **dadurch gekennzeichnet, dass** es wenigstens eine Nukleotidsequenz umfasst, die für ein Polypeptid codiert, das die Aminosäuren 34 bis 334 des durch die Sequenz SEQ ID NO:2 definierten Polypeptids umfasst.

**3.** Isoliertes Nukleinsäurefragment nach Anspruch 2, **dadurch gekennzeichnet, dass** die Sequenz den Nukleotiden 3836 bis 4738 der Sequenz SEQ ID NO:1 entspricht.

**4.** Isoliertes Nukleinsäurefragment nach irgendeinem der Ansprüche 1 bis 3, **dadurch gekennzeichnet, dass** es ferner eine Sequenz umfasst, die für ein Polypeptid codiert, das die Sekretion der Lipase ermöglicht.

**5.** Isoliertes Nukleinsäurefragment nach Anspruch 4, **dadurch gekennzeichnet, dass** es eine Sequenz umfasst, die für die Aminosäuren 1 bis 33 des durch die Sequenz SEQ ID NO:2 definierten Polypeptids codiert.

**6.** Isoliertes Nukleinsäurefragment nach Anspruch 5, **dadurch gekennzeichnet, dass** es die Sequenz umfasst, die den Nukleotiden 3737 bis 3835 der Sequenz SEQ ID NO:1 entspricht.

**7.** Expressionskassette für eine säureresistente extrazelluläre Lipase aus Hefe, **dadurch gekennzeichnet, dass** sie umfasst:

- ein Nukleinsäurefragment nach irgendeinem der Ansprüche 1 bis 6;
- Sequenzen, die es ermöglichen, die Expression der Lipase in einer Wirtszelle zu regulieren.

**8.** Rekombinanter Vektor, umfassend wenigstens ein Nukleinsäurefragment nach irgendeinem der Ansprüche 1 bis 6.

**9.** Rekombinanter Vektor nach Anspruch 8, umfassend wenigstens eine Expressionskassette nach Anspruch 7.

**10.** Eukaryontische oder prokaryontische Zelle, transformiert mit wenigstens einem Nukleinsäurefragment nach irgendeinem der Ansprüche 1 bis 6.

**11.** Transformierter Zellstamm nach Anspruch 10, **dadurch gekennzeichnet, dass** es sich um den *Yarrowia lipolytica*-Klon mit der Bezeichnung MS4 handelt, hinterlegt am 24. August 1999 bei der Collection Nationale de Culture de Microorganismes (C.N.C.M.) unter der Nummer I-2294.

**12.** Verfahren zur Herstellung einer säureresistenten Lipase, **dadurch gekennzeichnet, dass** es wenigstens einen Schritt umfasst, bei dem man transformierte Zellen nach irgendeinem der Ansprüche 10 oder 11 kultiviert, und einen Schritt, bei dem man die von diesen Zellen produzierte rekombinante Lipase erntet.

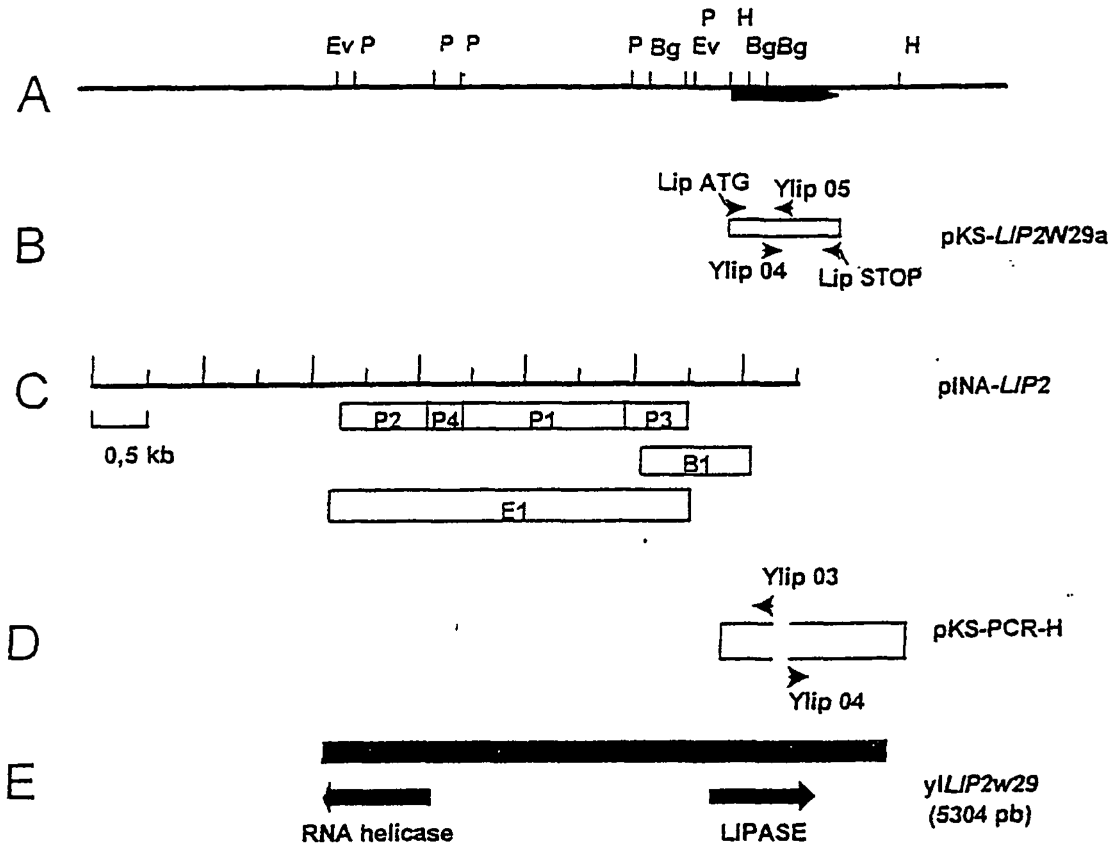
A
Ev P
P P
P Bg Ev
P H
BgBg
H
B
Lip ATG
Ylip 05
Ylip 04
Lip STOP
pKS-LIP2W29a
C
pINA-LIP2
0,5 kb
P2
P4
P1
P3
B1
E1
D
Ylip 03
Ylip 04
pKS-PCR-H
E
RNA helicase
LIPASE
ylLIP2w29
(5304 pb)

FIGURE 1

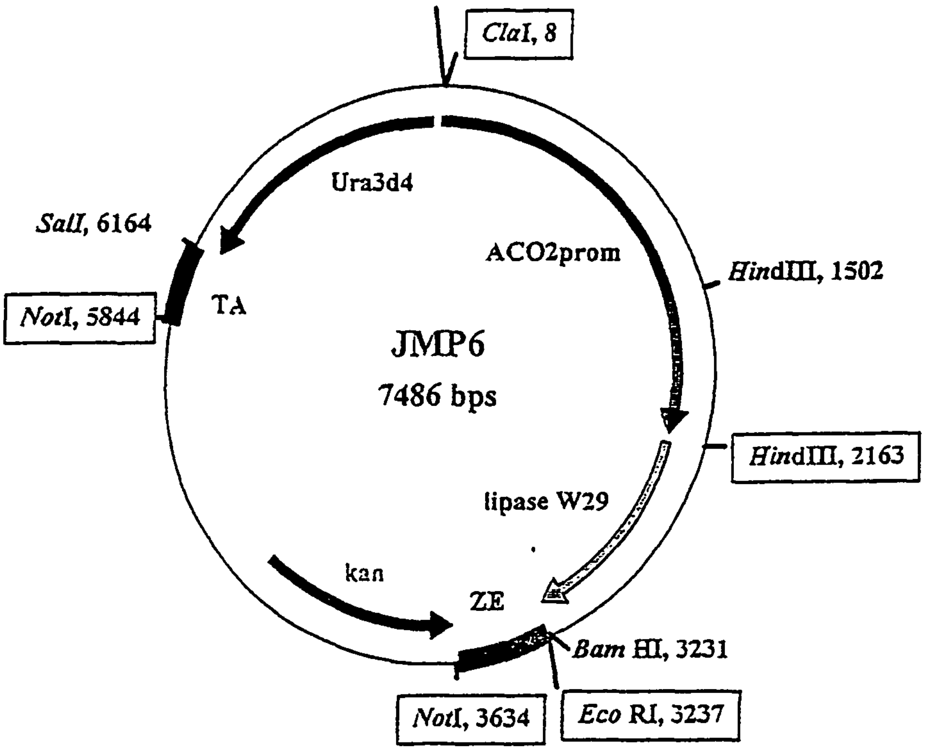
ClaI, 8
Ura3d4
SalI, 6164
ACO2prom
HindIII, 1502
NotI, 5844
TA
JMP6
7486 bps
HindIII, 2163
lipase W29
kan
ZE
Bam HI, 3231
NotI, 3634
Eco RI, 3237

FIGURE 2

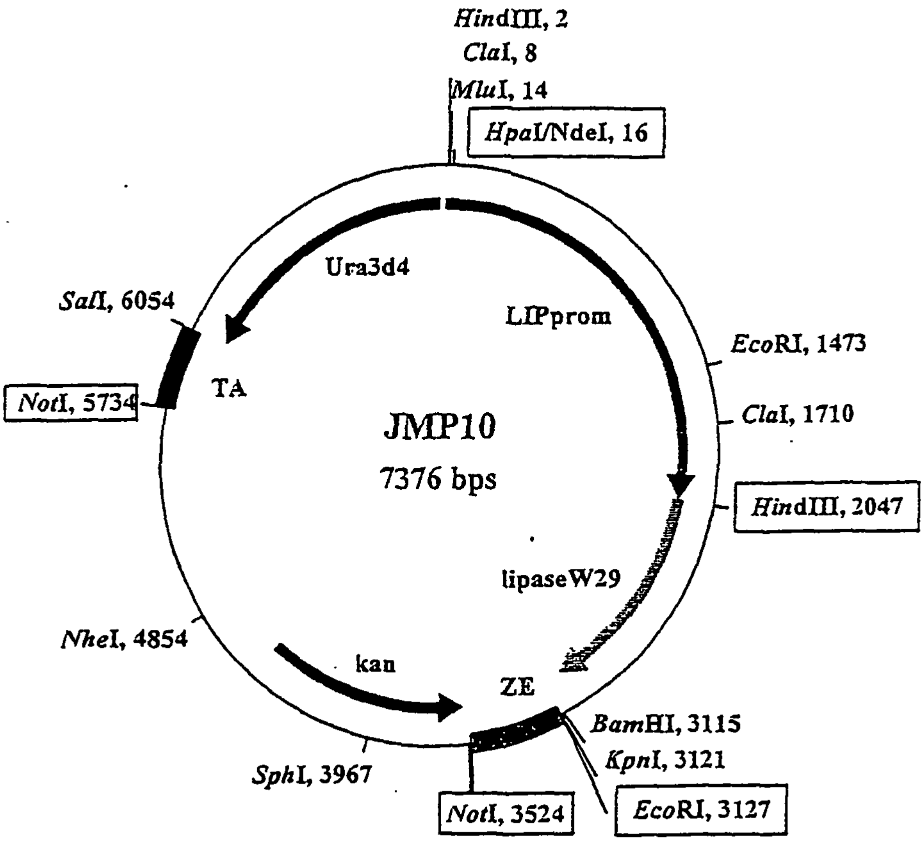
HindIII, 2
ClaI, 8
MluI, 14
HpaI/NdeI, 16
Ura3d4
LIPprom
EcoRI, 1473
ClaI, 1710
SalI, 6054
NotI, 5734
TA
JMP10
7376 bps
HindIII, 2047
lipaseW29
NheI, 4854
kan
ZE
BamHI, 3115
KpnI, 3121
EcoRI, 3127
SphI, 3967
NotI, 3524

FIGURE 3

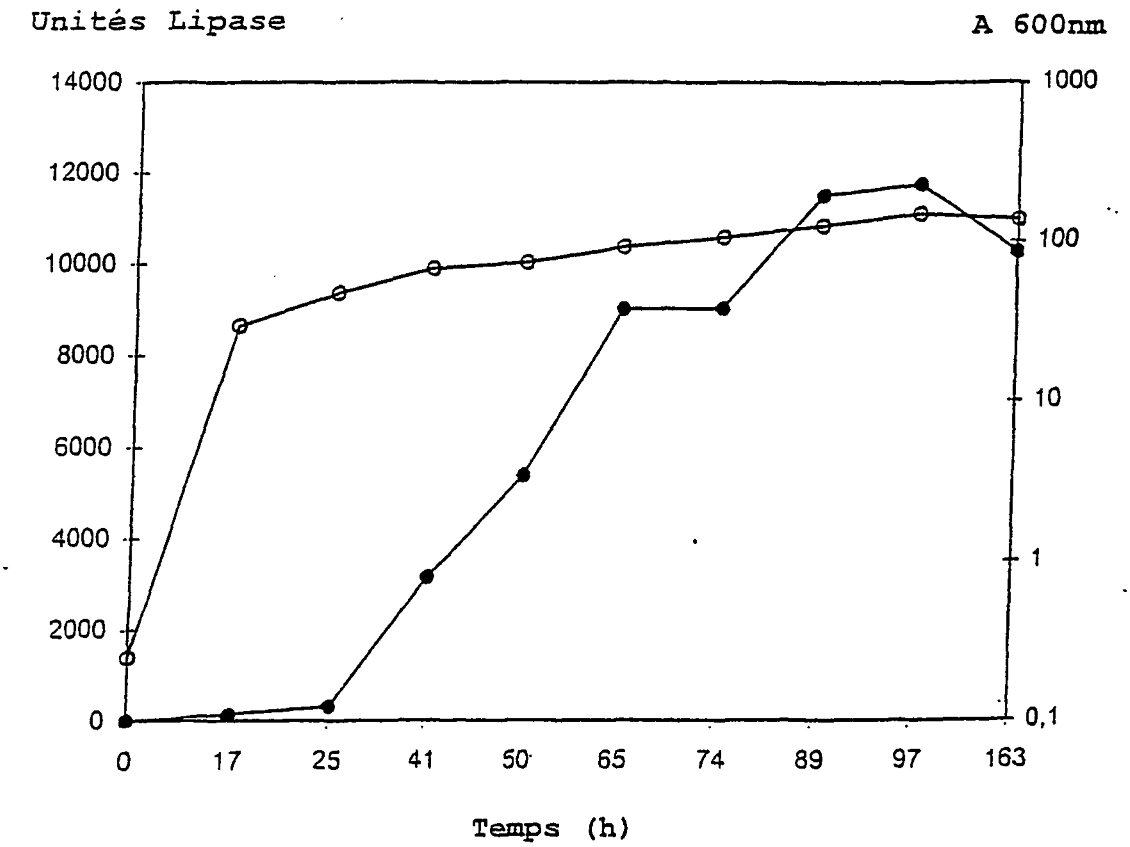
Unités Lipase
A 600nm
14000
12000
10000
8000
6000
4000
2000
0
1000
100
10
1
0,1
0
17
25
41
50
65
74
89
97
163
Temps (h)

FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0012729 A, l'INRA


### Littérature non-brevet citée dans la description


- **ZENTLER-MONRO et al.** *Pancreas,* 1992, vol. 7, 311-319
- **YOSHIDA et al.** *Biochim. Biophys. Acta,* 1968, vol. 154, 586-588
- **DHARMSTHITI et al.** *Biotechnol. Appl. Biochem.,* 1997, vol. 26, 111-116
- **UYTTENBROECK et al.** *Biol. Chem. Hoppe Seyler,* 1993, vol. 374, 245-254
- **HADEBALL.** *Acta Biotechnol.,* 1991, vol. 2, 159-167
- **NOVOTNY et al.** *J. Basic Microbiol.,* 1988, vol. 28, 221-227
- **XUAN et al.** *C. Curr. Genet.,* 1988, vol. 14, 15-21
- The dimorphic fungus Yarrowia lipolytica. **BARTH ; GAILLARDIN.** In: Non conventional yeasts in biotechnology. Springer-Verlag, 1996, 313-388